# FASCICULE DE BREVET EUROPEEN

(11) **EP 3 160 650 B1**
(45) Date de publication et mention de la délivrance du brevet: **01.08.2018**
(21) Numéro de dépôt: 15753079.1
(22) Date de dépôt: 23.06.2015
(51) Int. Cl.: B02C 17/16, B02C 17/18

(54) **ARBRE D'AGITATION POUR BROYEUR**
RÜHRERSCHAFT FÜR EINE MÜHLE
AGITATOR SHAFT FOR A GRINDING MILL

(30) Priorité: 25.06.2014 FR 1455925
(43) Date de publication de la demande: 03.05.2017
(73) Titulaire: Corbion Biotech, Inc., South San Francisco, CA 94080 (US)
(72) Inventeur: SEGARD, David, F-62136 Lestrem (FR); PATINIER, Samuel, F-59890 Quesnoy sur Deule (FR)
(74) Mandataire: Gallois, Valérie
(86) Numéro de dépôt international: PCT/FR2015/051684
(87) Numéro de publication internationale: WO 2015/197972

(56) Documents cités:
- WO-A2-2014/005570
- GB-A- 2 268 426
- US-A- 5 882 246

## Description

L'invention concerne un arbre d'agitation, ainsi qu'un broyeur équipé d'un tel arbre d'agitation. Un tel arbre d'agitation et broyeur trouveront une application particulière pour la lyse de biomasses de microorganismes produites à l'échelle industrielle, notamment par fermentation, telles que les micro-algues, plus particulièrement les microalgues du genre *Chlorella.*

Le domaine de l'invention est celui des broyeurs à billes qui comprennent classiquement une enceinte de traitement définie par le volume intérieur d'une cuve, typiquement cylindrique, à l'intérieur duquel peuvent être broyées des matières, telles que des microorganismes cellulaires par voie sèche, ou en milieu liquide. La lyse des microorganismes unicellulaires est obtenue par l'effet des forces de cisaillement résultant du broyage et de la collision entre elles des petites billes, intérieures à l'enceinte, qui sont mises en mouvement par un arbre d'agitation du broyeur, entrainé en rotation sur son axe, sous l'action d'un motoréducteur.

Les documents US 3.844.490, US. 5.785.262, US2005/0011976 ou encore WO 2014/005570 enseignent de tels broyeurs à billes, à axe horizontal ou à axe vertical. L'arbre d'agitation d'un broyeur à billes comprend classiquement, et tel qu'illustré à la vue de coupe de la figure 1 du document WO2014/005570, un axe central le long duquel sont répartis des éléments d'agitation, globalement plans, et sensiblement perpendiculaires à l'axe, que nous nommerons par la suite « *disques d'agitation* », dans un souci de simplification. Il est connu d'espacer ces disques d'agitation les uns par rapport aux autres grâce à des entretoises tubulaires, montées autour de l'axe, et prenant appui chacune à leurs extrémités sur deux disques d'agitation successifs de l'arbre.

L'assemblage d'un tel arbre d'agitation consiste essentiellement à enfiler successivement, et en alternance, les entretoises et les disques d'agitation sur l'axe central jusqu'à leur mise en contact. L'empilement des entretoises et des disques d'agitation est maintenu entre deux butées extrêmes solidaires de l'axe central et dont l'écartement est maintenu par un système de serrage. Usuellement, des baguettes (ou joncs en acier) traversent des gorges des disques d'agitation, sur le diamètre interne, et dans le but de solidariser en rotation l'axe et les disques d'agitation.

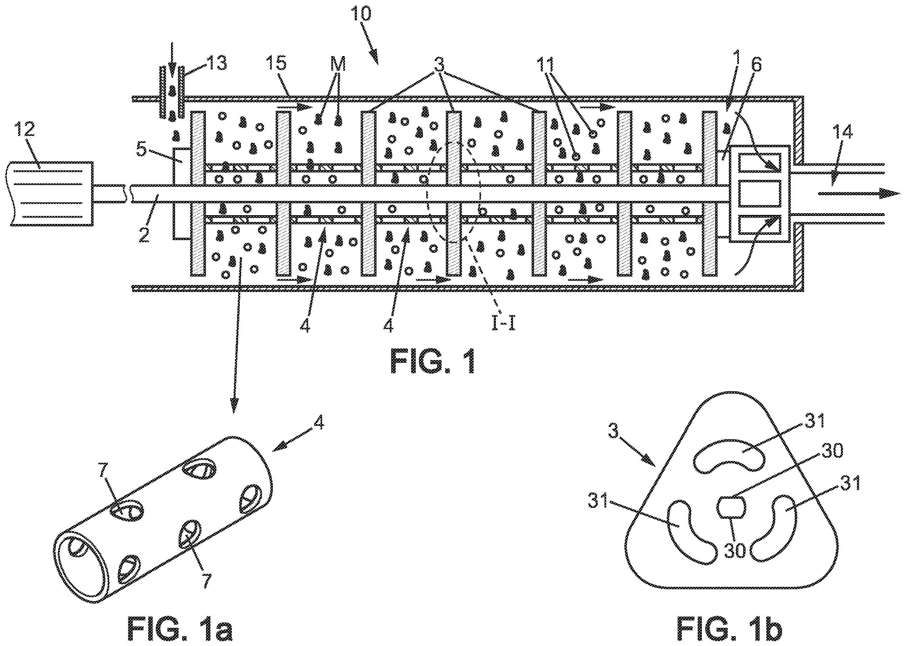

Par exemple, et dans le document US 2005/0011976 A1, à la figure 12, les disques d'agitation sont repérés 22, l'axe central repéré 24 et les entretoises, dénommées *« spacer* », repérées 20.
Comme visible à la vue de coupe de la figure 1 du document WO 2014/005570, l'arbre d'agitation comprend un volume interstitiel libre, défini entre la surface extérieure de l'axe central et les parois intérieures des entretoises tubulaires, des disques d'agitation, ainsi qu'une flasque d'extrémité (à droite sur la figure 1). Comme visible sur cette même figure, les billes sont disposées dans l'enceinte de traitement, avec les matières à broyer, dans le volume de traitement défini entre la paroi intérieure de la cuve et la surface extérieure de l'arbre d'agitation.

En revanche, l'arbre d'agitation est rendu étanche, afin d'interdire toute insertion de matière et des billes dans le creux de l'arbre d'agitation. En particulier, l'assemblage entre les divers éléments (entretoises, disques d'agitation, axe central, flasque) est un assemblage étanche : les entretoises sont tubulaires, de parois pleines, l'étanchéité aux fluides étant complétée par des joints entre les surfaces d'appui entretoises/disques d'agitation, joints qui sont comprimés lors du serrage. Des rondelles d'étanchéité (en cuivre) sont associées aux vis utilisées pour le serrage du flasque d'extrémité afin d'interdire les fuites le long de ces dernières, au travers du flasque.

La présente Demanderesse a conduit des essais d'utilisation d'un tel broyeur à billes. En particulier, le but recherché était de lyser des microorganismes unicellulaires (micro-algues de type chlorelles), cultivés dans un bioréacteur, présentant une concentration de 300 grammes à 400 grammes (en matière sèche) par litre. Les micro-algues, en milieu liquide, sont acheminées, selon un débit constant, au niveau de l'ouverture d'alimentation du broyeur à billes, à l'une des extrémités de l'enceinte. Dans l'enceinte de traitement, ces micro-algues sont lysées, par le travail des billes qui sont remuées par l'arbre d'agitation, entrainé sur son axe à une vitesse proche de 1000 tr/min, les cellules broyées ressortant en continu à l'autre extrémité de l'enceinte.

Les microalgues de type chlorelles sont des organismes extrêmement sensibles aux contaminations d'origine notamment bactérienne ; il est impératif de conserver des conditions opérationnelles satisfaisantes de manière à éviter toute contamination lors des opérations de broyage. L'apparition d'une contamination est rédhibitoire et implique de mettre le contenu au rebus, puis de stériliser l'installation, avant la mise en oeuvre d'une nouvelle production. Les contaminations provoquent ainsi des arrêts de production conséquents.

Sur ce point, et d'après les constatations de l'inventeur, la conception de l'arbre d'agitation tel que connu de l'état de la technique est à risque, en ce qu'un défaut d'étanchéité de l'arbre autoriserait une insertion de matière dans le creux de l'arbre, stagnante, favorisant ainsi l'apparition d'une contamination, interne à l'arbre, qui en raison de ce même défaut se propagerait, en retour, aux matières dans l'enceinte de traitement.

Lors des tests, la présente Demanderesse a donc examiné avec soin l'étanchéité de l'arbre d'agitation. En particulier, et dans les conditions ci-dessus mentionnées il est apparu que l'arbre d'agitation n'était pas parfaitement étanche.

Le but de la présente invention est de proposer un arbre d'agitation pour broyeur à billes qui pallie les inconvénients précités, en particulier qui supprime les risques de contaminations tels qu'ils peuvent être rencontrés dans les arbres d'agitation de l'état de la technique.

Un autre but de la présente invention est de proposer un tel arbre d'agitation requérant, en utilisation, une maintenance simplifiée.

D'autres buts et avantages de la présente invention apparaîtront au cours de la description qui va suivre qui n'est donnée qu'à titre indicatif et qui n'a pas pour but de la limiter.

L'invention concerne tout d'abord un arbre d'agitation pour broyeur comprenant :
- un axe destiné à être entraîné en rotation,
- une succession d'éléments d'agitation, plans, et d'entretoises, tubulaires, montés en alternance sur l'axe, suivant un empilement le long dudit axe,
- des butées extrêmes, solidaires de l'axe, maintenant en compression l'empilement d'éléments d'agitation et d'entretoises.

Selon l'invention, les entretoises tubulaires présentent des orifices autorisant la libre circulation des fluides et de la matière, de l'extérieur vers l'intérieur de l'arbre d'agitation, et inversement.

Selon des caractéristiques optionnelles de l'invention, prises seules ou en combinaison :
- les entretoises tubulaires sont des éléments monoblocs ;
- les orifices sont de diamètres compris entre 4 mm et 50 mm, de préférence entre 20 mm et 30 mm ;
- les orifices sont polis, voire préalablement chanfreinés;
- les éléments d'agitation sont liés en rotation à l'axe grâce à des méplats entre l'axe et les éléments d'agitation, ou alternativement par des joncs d'acier ;
- l'axe est dans un acier inox 1.4418 ou X4CrNiMo16.5.1 selon le standard Euronorm ;
- les éléments d'agitation comprennent chacun, une plaque, pourvue d'un orifice central pour le passage de l'axe, sensiblement perpendiculaire à l'axe, et de préférence des évidements traversant répartis autour dudit axe ;
- les entretoises appuient sur les faces des éléments d'agitation par leurs extrémités, par l'intermédiaire de joints entre les faces d'appui des entretoises, d'une part, et les faces d'appui des éléments d'agitation, d'autre part.

L'invention concerne également un broyeur à billes comprenant une cuve recevant un arbre d'agitation conforme à l'invention. De préférence, les orifices des entretoises sont dimensionnés supérieurs à la taille des billes de manière à autoriser la libre circulation des billes de l'extérieur vers l'intérieur de l'arbre d'agitation, et inversement.

L'invention sera mieux comprise à la lecture de la description suivante accompagnée des dessins en annexe parmi lesquels :
- La figure 1 est une vue schématique, selon un plan de coupe passant par l'axe, d'un broyeur à billes conforme à l'invention, équipé d'un arbre d'agitation selon l'invention.
- La figure la est une vue de détail, en perspective, d'une entretoise ajourée conforme à l'invention, équipant l'arbre d'agitation du broyeur de la figure 1.
- La figure 1b est une vue de détail, de face, d'un élément d'agitation équipant l'arbre d'agitation du broyeur de la figure 1.
- La figure 2 est une vue de détail du cadre I-I tel qu'illustré à la figure 1.
- La figure 3 est une vue schématique illustrant l'assujettissement en rotation entre l'axe et un des éléments d'agitation par l'intermédiaire de méplats,

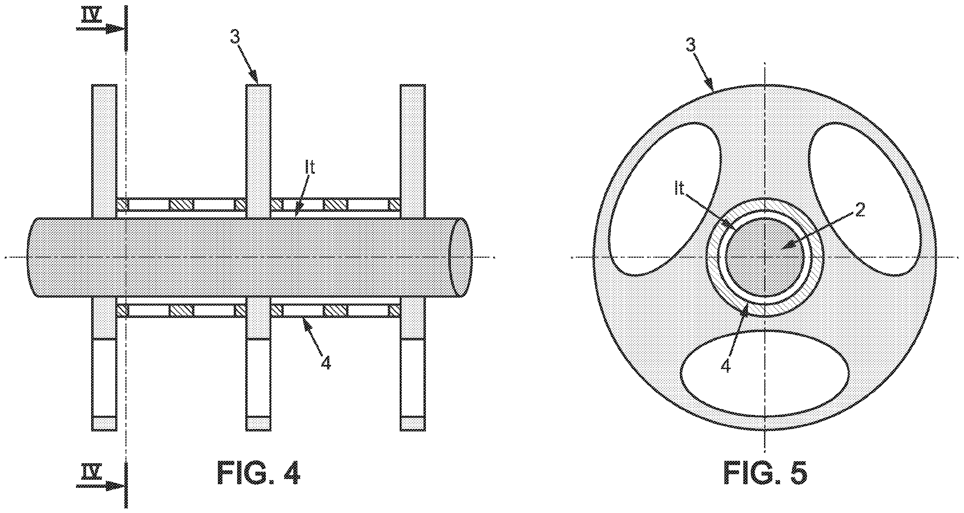

- La figure 4 est une vue en transparence d'une section de longueur de l'arbre d'agitation,
- La figure 5 est une vue selon la coupe IV-IV telle qu'illustrée à la figure 4,

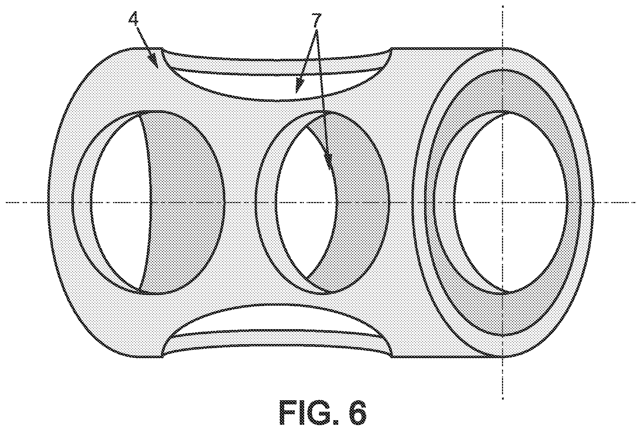

- La figure 6 est une vue de détail de l'entretoise de la figure 4,

L'invention concerne tout d'abord un arbre d'agitation 1 pour broyeur comprenant :
- un axe 2 destiné à être entraîné en rotation, typiquement par un motoréducteur 12,
- une succession d'éléments d'agitation 3, plans, et d'entretoises 4, tubulaires, montés en alternance sur l'axe, suivant un empilement le long dudit axe 2,
- des butées extrêmes 5,6, solidaires de l'axe, maintenant en compression l'empilement d'éléments d'agitation 3 et d'entretoises 7.

Un tel assemblage est illustré à titre d'exemple non limitatif à la figure 1. L'assemblage d'un tel arbre d'agitation consiste essentiellement à enfiler successivement, et en alternance, les entretoises 4 et les éléments d'agitation 3 sur l'axe 2, central, jusqu'à leur mise en contact. L'empilement des entretoises 4 et des éléments d'agitation 3 est maintenu entre les deux butées extrêmes, gauche, repérée 5 et droite repérée 6, solidaires de l'axe central et dont l'écartement est maintenu par un système de serrage. Les éléments d'agitation 3 peuvent comprendre chacun une plaque, pourvue d'un orifice central pour le passage de l'axe 2, voire de préférence des évidements 31 traversant répartis autour de l'axe. Chacun des éléments d'agitation peut être de contour non circulaire, tel qu'illustré à la figure 1b, les éléments d'agitation 3 étant de préférence alors décalés angulairement, en alternance, suivant la longueur de l'axe 2.

Une telle conception d'arbre d'agitation est connue de l'état de la technique, en particulier du document WO 2014/005570. Dans un tel état de la technique connu de la présente Demanderesse, l'arbre d'agitation est un arbre étanche de manière à interdire une insertion de matière, depuis l'enceinte de traitement jusque dans le creux de l'arbre d'agitation.

Les tests réalisés par la présente Demanderesse, dans les conditions mentionnées dans l'introduction, ont toutefois démontré que cette étanchéité n'était pas parfaite, ce défaut d'étanchéité constituant un risque d'apparition de contamination.

Selon la compréhension actuelle de l'inventeur, un tel défaut d'étanchéité s'expliquerait, lors des tests réalisés, par la forte charge qui s'exercerait sur l'arbre d'agitation, en dynamique, provoquant des contraintes (flexion et/ou de torsion) à l'origine d'une perte d'étanchéité.

Selon les réflexions de l'inventeur, et naturellement, il serait possible de renforcer l'étanchéité de l'arbre par une conception améliorée de l'arbre d'agitation, par exemple en augmentant le nombre de joints et leur qualité. Toutefois, et selon l'expérience de l'inventeur, l'étanchéité d'un tel arbre d'agitation devrait toujours être contrôlée périodiquement, et impliquerait nécessairement des opérations de maintenance périodiques, lourdes et couteuses, telles que des changements de joints.

L'invention est née de la constatation par l'inventeur que la mise en oeuvre d'un arbre d'agitation étanche est problématique.

La solution proposée par l'inventeur est à contre-pied de ce que ferait l'homme du métier : puisqu'il apparaît difficile d'assurer une étanchéité parfaite de l'arbre d'agitation, la solution proposée par l'inventeur est au contraire de prévoir des entretoises, largement perméables, de manière à assurer l'évacuation, la vidange et le nettoyage du volume intérieur libre à l'entretoise.

L'objectif poursuivi ici est donc de supprimer dans l'arbre d'agitation les cavités internes aux entretoises dans lesquelles la matière et les fluides peuvent s'insérer puis stagner, en contaminant en retour la chambre de broyage en retour.

Selon l'invention, les entretoises tubulaires 3 présentent avantageusement des orifices 7 autorisant la libre circulation des fluides et de la matière de l'extérieur vers l'intérieur de l'arbre d'agitation 1, et inversement. Les orifices 7 sont de diamètres compris entre 4 mm et 50 mm, de préférence entre 20 mm et 30 mm.

De préférence, ces orifices 7 sont dimensionnés supérieurs à la dimension des billes 11 utilisées dans le broyeur 10. Le libre passage des billes au travers de l'entretoise 4, permet, lors du fonctionnement, d'assurer une meilleure nettoyabilité des cavités internes (interstice It) lors du fonctionnement.

De préférence, la géométrie des orifices 7 est conçue de manière à favoriser une circulation des fluides et de la matière à broyer, lors de la rotation de l'arbre d'agitation, de l'extérieur vers l'intérieur de l'entretoise, et inversement.

Les entretoises tubulaires 4 peuvent être des éléments monoblocs, par exemple cylindriques. Les orifices peuvent être réalisés par usinage de la paroi de l'entretoise tubulaire. De préférence, ces orifices 7 sont polis, voire chanfreinés, dans le but de limiter au maximum les dépôts, et ainsi l'apparition et le développement de contamination.

Selon les constatations de l'inventeur, cette nouvelle conception d'arbre d'agitation, perméable aux fluides et aux matières à broyer et, de préférence aux billes de broyage, présente toutefois le défaut d'exposer l'axe central 2 à des contraintes plus fortes que celles rencontrées par un axe central d'un arbre d'agitation étanche, tel que connu de l'état de la technique. L'axe d'un arbre d'agitation selon l'état de la technique est en effet protégé des matières à broyer et des billes de broyage, et ainsi soumis à des risques de corrosion plus faible.

Afin de palier cette contrariété, le choix du matériau de l'axe 2 est important en ce qu'il détermine non seulement sa résistance mécanique en dynamique, en particulier à la torsion et à la flexion, mais également sa résistance à la corrosion. De manière à augmenter la durée de vie de l'équipement, selon l'invention, on peut avantageusement choisir un acier inoxydable dont les performances mécaniques et les performances à la corrosion répondent à ces nouvelles contraintes, plus exigeantes. A cet effet, l'axe 2 peut être dans un acier inox 1.4418 ou X4CrNiMo16.5.1 (ou un acier martensitique) selon le standard Euronorm.

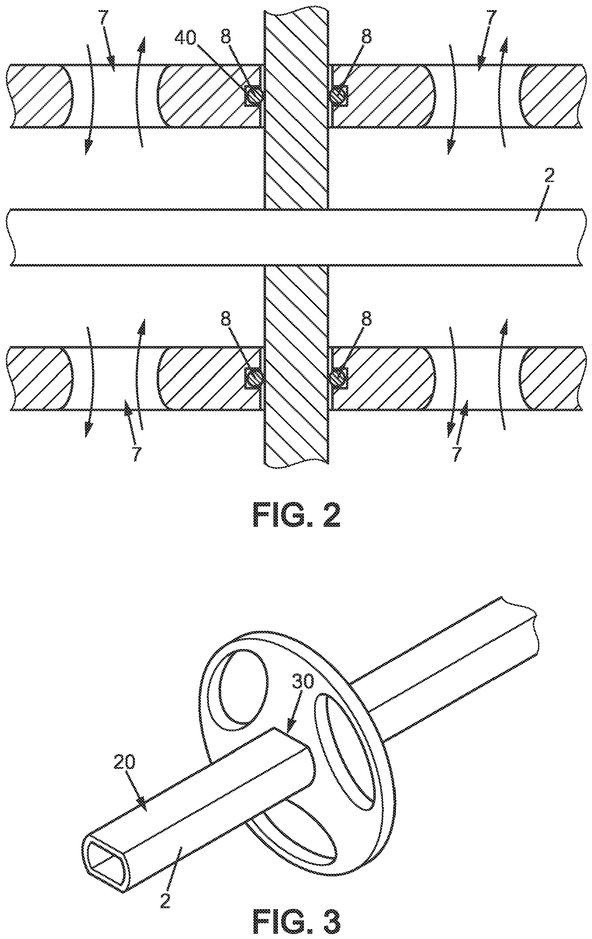

Selon un mode de réalisation, illustré non limitativement à la figure 3, les éléments d'agitation 3 peuvent être liés en rotation à l'axe grâce à des méplats 20, 30 entre l'axe 2 et les éléments d'agitation 3. Dans ce cas, l'orifice central des éléments d'agitation permet l'insertion de l'axe 2 au jeu de fonctionnement près. Ces méplats 20 et 30 entre l'axe 2 et l'élément d'agitation 3 coopèrent afin d'interdire un mouvement de rotation relatif entre ces deux éléments. Alternativement, des joncs acier peuvent être utilisés.

Selon un mode de réalisation non illustré, les entretoises 7 peuvent appuyer directement (*i.e.* sans joints) sur les faces des éléments d'agitation 3 par leurs extrémités. Selon une alternative de réalisation, illustrée non limitativement à la figure 2, les entretoises 7 appuient sur les faces des éléments d'agitation 3 par leurs extrémités, par l'intermédiaire de joints 8 entre, les faces d'appui des entretoises 4, d'une part, et les faces d'appui des éléments d'agitation 3, d'autre part. Dans un tel cas, l'objectif visé par ces joints 8 n'est évidemment pas d'assurer une étanchéité au fluide, puisque les entretoises sont perméables aux liquides. Ces joints 8 permettant notamment de faciliter l'assemblage, en étant plus tolérant sur les dimensions de fabrication des divers, à savoir les éléments d'agitation et des entretoises. Ces joints 8 peuvent permettre de limiter les risques de marquage des faces des éléments d'agitation, au niveau de la surface d'appui des entretoises 4. Afin d'éviter un écrasement des joints 8 lors du serrage, ces derniers peuvent être reçus partiellement dans des gorges 40 en profondeur des surfaces d'appui des entretoises 7. Les joints 8 sont des joints circulaires, de préférence annulaires.

L'invention concerne également un broyeur à billes 10 comprenant une cuve 15 recevant un arbre d'agitation 1 conforme à l'invention. De préférence, les orifices 7 des entretoises 4 sont dimensionnés supérieurs à la taille des billes 11 de manière à autoriser la libre circulation des billes de l'extérieur vers l'intérieur de l'arbre d'agitation 1, et inversement lors du fonctionnement. Le broyeur est de préférence à axe horizontal.

Il s'agit de préférence d'un broyeur assurant un traitement des matières, repérées M, en continu. Le broyeur présente une entrée 13 pour la matière à broyer et une sortie 14 pour le broyât. Un tel broyeur est de préférence alimenté en continu, selon un débit déterminé.

L'invention trouve une application particulière comme broyeur industriel. Le débit d'alimentation au broyeur peut être compris entre 0.5 m³/h à 10 m³/h et pour des concentrations de matières (en matière sèche) de 300 g/l à 400g/l, conditions pour lesquelles l'arbre d'agitation 1 est soumis à de fortes contraintes mécaniques.

La maintenance du broyeur peut prévoir, notamment entre deux phases de production, le nettoyage des parois internes de la cuve 15, des parois internes et externes de l'arbre d'agitation 1, ainsi que de l'axe 2, en alimentant le broyeur d'une solution de nettoyage. Lors de cette étape, l'arbre d'agitation 1 est de préférence animé d'un mouvement de rotation afin de favoriser la circulation de la solution de nettoyage au travers de l'arbre d'agitation 1, par l'intermédiaire des orifices 7.

Naturellement d'autres modes de réalisation auraient pu être envisagés par l'homme du métier sans pour autant sortir du cadre de l'invention tel que définie ci-après.

### NOMENCLATURE

1. Arbre d'agitation,
2. Axe,
3. Elément d'agitation,
4. Entretoises tubulaires,
5,6. Butées extrêmes,
7. Orifices (Entretoises),
8. Joint,
10. Broyeur à billes,
11. Billes,
12. Motoréducteur,
13. Entrée matière (Broyeur),
14. Sortie matière (Broyeur),
15. Cuve,
20. Méplats (Axe),
30. Méplats (Eléments d'agitation),
31. Evidements (Eléments d'agitation),
40. Gorges (pour joint)
M. Matière,
It. Interstice (entre axe et entretoise).

## Revendications

1. Arbre d'agitation (1) pour broyeur comprenant :
- un axe (2) destiné à être entraîné en rotation,
- une succession d'éléments d'agitation (3), plans, et d'entretoises (4), tubulaires, montés en alternance sur l'axe, suivant un empilement le long dudit axe (2),
- des butées extrêmes (5,6), solidaires de l'axe, maintenant en compression l'empilement d'éléments d'agitation et d'entretoises,
**caractérisé en ce que** les entretoises tubulaires (4) présentent des orifices (7) autorisant la libre circulation de la matière et des fluides de l'extérieur vers l'intérieur de l'arbre d'agitation, et inversement.

2. Arbre d'agitation selon la revendication 1, dans lequel les entretoises tubulaires (4) sont des éléments monoblocs.

3. Arbre d'agitation selon la revendication 1 ou 2, dans lequel les orifices (7) sont de diamètres compris entre 4 mm et 50 mm, de préférence entre 20 mm et 30 mm.

4. Arbre d'agitation selon l'une des revendications 1 à 3, dans lequel les orifices (7) sont polis voire chanfreinés.

5. Arbre d'agitation selon l'une des revendications 1 à 4 dans lequel les éléments d'agitation (3) sont liés en rotation à l'axe grâce à des méplats (20, 30) entre l'axe (2) et les éléments d'agitation (3).

6. Arbre d'agitation selon l'une des revendications 1 à 5 dans lequel l'axe (2) est dans un acier inox 1.4418 ou X4CrNiMo16.5.1 selon le standard Euronorm.

7. Arbre d'agitation selon l'une des revendications 1 à 6 dans lequel les éléments d'agitation (3) comprennent chacun, une plaque, pourvue d'un orifice central pour le passage de l'axe, sensiblement perpendiculaire à l'axe.

8. Arbre d'agitation selon l'une des revendications 1 à 7 dans lequel les entretoises (4) appuient sur les faces des éléments d'agitation (3) par leurs extrémités, par l'intermédiaire de joints (8) entre, les faces d'appui des entretoises (4), d'une part, et les faces d'appui des éléments d'agitation (3), d'autre part.

9. Broyeur (10) à billes comprenant une cuve (15) recevant un arbre d'agitation (1) selon l'une des revendications 1 à 8.

10. Broyeur selon la revendication 9, dans lequel les orifices (7) des entretoises (4) sont dimensionnés supérieurs à la taille de billes (11) de manière à autoriser la libre circulation des billes de l'extérieur vers l'intérieur de l'arbre d'agitation (1), et inversement.

11. Procédé de nettoyage d'un broyeur selon la revendication 9 ou 10, dans lequel on nettoie les parois internes de la cuve (15), les parois internes et externes de l'arbre d'agitation (1), en alimentant le broyeur d'une solution de nettoyage, et de préférence en animant l'arbre d'agitation (1) en rotation et de manière à ce que la solution de nettoyage circule au travers de l'arbre d'agitation (1).

12. Utilisation d'un arbre d'agitation (1) selon l'une des revendications 1 à 8, ou d'un broyeur selon la revendication 9 ou 10 pour le broyage de microorganismes unicellulaires, par exemple de microalgues de type chlorelles.

## Patentansprüche

1. Rührerschaft (1) für eine Mühle, umfassend:
- eine Achswelle (2), die in Rotation versetzt werden soll,
- eine Abfolge von flachen Rührelementen (3) und röhrenförmigen Abstandshaltern (4), die auf der Achswelle und als Stapel entlang besagter Achswelle (2) alternierend montiert sind,
- Endstopper (5, 6), die fest mit der Achswelle verbunden sind und den Stapel aus Rührelementen und Abstandshalter zusammendrücken,
**dadurch gekennzeichnet, dass** die röhrenförmigen Abstandshalter (4) Öffnungen (7) aufweisen, die die freie Zirkulation von Materie und Flüssigkeiten von außen nach innen des Rührerschafts und umgekehrt zulassen.

2. Rührerschaft gemäß Anspruch 1, wobei die röhrenförmigen Abstandshalter (4) einstückige Elemente sind.

3. Rührerschaft gemäß Anspruch 1 oder 2, wobei die Öffnungen (7) einen Durchmesser zwischen 4 mm und 50 mm, vorzugsweise zwischen 20 mm und 30 mm, besitzen.

4. Rührerschaft gemäß einem der Ansprüche 1 bis 3, wobei die Öffnungen (7) glatt oder abgeschrägt sind.

5. Rührerschaft gemäß einem der Ansprüche 1 bis 4, wobei die Rührelemente (3) drehbar mit der Achswelle durch Abflachungen (20, 30) zwischen der Achswelle (2) und den Rührelementen (3) verbunden sind.

6. Rührerschaft gemäß einem der Ansprüche 1 bis 5, wobei die Achswelle (2) aus Edelstahl 1.4418 oder X4CrNiMo16.5.1 gemäß Euronorm Standard hergestellt ist.

7. Rührerschaft gemäß einem der Ansprüche 1 bis 6, wobei die Rührelemente (3) jeweils eine Platte, die mit einer zentralen Öffnung für den Durchtritt der Achswelle versehen ist, im Wesentlichen senkrecht zur Achswelle umfassen.

8. Rührerschaft gemäß einem der Ansprüche 1 bis 7, wobei die Abstandshalter gegen die Seiten der Rührelemente (3) mit ihren Enden über Dichtungen (8) zwischen den Auflageflächen der Abstandshalter (4) einerseits und den Auflageflächen der Rührelemente (3) andererseits drücken.

9. Kugelmühle (10), umfassend einen Behälter, der einen Rührerschaft (1) gemäß einem der Ansprüche 1 bis 8 aufnimmt.

10. Mühle gemäß Anspruch 9, wobei die Öffnungen (7) der Abstandshalter (4) größer als die Größe der Kugeln (11) bemessen sind, um die freie Zirkulation der Kugeln von außen nach innen des Rührerschafts (1) und umgekehrt zuzulassen.

11. Verfahren zur Reinigung einer Mühle gemäß Anspruch 9 oder 10, wobei die Innenwände des Behälters (15), die Innen- und Außenwände des Rührerschafts (1) durch Zufuhr einer Reinigungslösung in die Mühle gereinigt werden, und vorzugsweise der Rührerschaft (1) in Rotation versetzt wird, damit die Reinigungslösung durch den Rührerschaft (1) fließt.

12. Verwendung eines Rührerschafts (1) gemäß einem der Ansprüche 1 bis 8 oder einer Mühle gemäß Anspruch 9 oder 10 zur Zerkleinerung einzelliger Mikroorganismen, zum Beispiel Mikroalgen des Typs Chlorella.

## Claims

1. An agitator shaft (1) for a mill, comprising:
- a spindle (2) intended to be driven in rotation,
- a series of flat agitator elements (3) and tubular spacers (4) that are mounted in alternation on the spindle and in a stack along said spindle (2),
- end stops (5, 6) that are secured to the spindle and keep the stack of agitator elements and spacers under compression,
**characterized in that** the tubular spacers (4) have orifices (7) that allow the matter and fluids to flow freely from the outside of the agitator shaft to the inside, and vice versa.

2. The agitator shaft as claimed in claim 1, wherein the tubular spacers (4) are one-piece elements.

3. The agitator shaft as claimed in claim 1 or 2, wherein the orifices (7) have diameters of between 4 mm and 50 mm, preferably between 20 mm and 30 mm.

4. The agitator shaft as claimed in one of claims 1 to 3, wherein the orifices (7) are smooth or even chamfered.

5. The agitator shaft as claimed in one of claims 1 to 4, wherein the agitator elements (3) are rotationally linked to the spindle by virtue of flats (20, 30) between the spindle (2) and the agitator elements (3).

6. The agitator shaft as claimed in one of claims 1 to 5, wherein the spindle (2) is made of stainless steel grade 1.4418 or X4CrNiMo16.5.1 according to the Euronorm standard.

7. The agitator shaft as claimed in one of claims 1 to 6, wherein the agitator elements (3) each comprise a plate provided with a central orifice for the passage of the spindle, substantially perpendicular to the spindle.

8. The agitator shaft as claimed in one of claims 1 to 7, wherein the spacers (4) bear against the faces of the agitator elements (3) with their ends, by way of seals (8) between the bearing faces of the spacers (4), for the one part, and the bearing faces of the agitator elements (3), for the other part.

9. A bead mill (10) comprising a tank (15) that holds an agitator shaft (1) as claimed in one of claims 1 to 8.

10. The mill as claimed in claim 9, wherein the orifices (7) in the spacers (4) have a larger size than that of the beads (11) so as to allow the beads to flow freely from the outside of the agitator shaft (1) to the inside, and vice versa.

11. A method for cleaning a mill as claimed in claim 9 or 10, wherein the internal walls of the tank (15) and the internal and external walls of the agitator shaft (1) are cleaned by supplying the mill with a cleaning solution, and preferably by setting the agitator shaft (1) in rotation such that the cleaning solution flows through the agitator shaft (1).

12. The use of an agitator shaft (1) as claimed in one of claims 1 to 8, or of a mill as claimed in claim 9 or 10 for grinding single-cell microorganisms, for example microalgae of the chlorella type.
